(19) Europäisches Patentamt
European Patent Office
Office européen des brevets

(11) Veröffentlichungsnummer: 0 337 179
A1

(12) **EUROPÄISCHE PATENTANMELDUNG**

(21) Anmeldenummer: 89105352.2

(51) Int. Cl.4: C07D 405/04 , A61K 31/535

(22) Anmeldetag: 25.03.89

Patentanspruch für folgenden Vertragsstaat: ES + GR

(30) Priorität: 31.03.88 DE 3811017

(43) Veröffentlichungstag der Anmeldung: 18.10.89 Patentblatt 89/42

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Englert, Heinrich Christian, Dr.
Stormstrasse 13
D-6238 Hofheim am Taunus(DE)
Erfinder: Mania, Dieter, Dr.
Berliner Ring 5
D-6233 Kelkheim (Taunus)(DE)
Erfinder: Schölkens, Bernward, Dr.
Hölderlinstrasse 62
D-6233 Kelkheim (Taunus)(DE)
Erfinder: Utz, Roland, Dr.
Sudetenstrasse 18
D-6101 Messel(DE)

(54) **Ungesättigte N-Benzopyranyllactame.**

(57) Beschrieben werden Verbindungen I

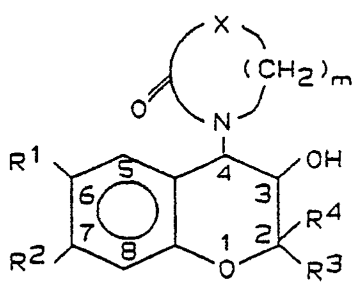

I

mit $R^1$ gleich H, Alkyl, Alkoxy, CO Alkyl, COOH, Carboxyalkyl, $CONR_2$, CN, $NO_2$, Alkylsulfi(o)nyl, Arylsulfi(o)nyl; $R^2$ gleich H, OH, Alkoxy, Alkyl, Alkylcarbonyl, $R^3$/$R^4$ gleich Alkyl, m gleich null oder eins, X gleich $-CR^6=CR^7-(-CR^8=CR^9-)-_n$ ($R^6$ bis $R^9$ gleich H, Alkyl) mit n gleich null oder 1. Sie sind wirksame Antihypertensiva und Spasmolytika für Blase, Darm, Galle, Uterus, Trachea und Ureter.

EP 0 337 179 A1

## Ungesättigte N-Benzopyranyllactame

Die Erfindung betrifft ungesättigte 3,4-Dihydro-2H-benzo[b]pyrane der Formel I

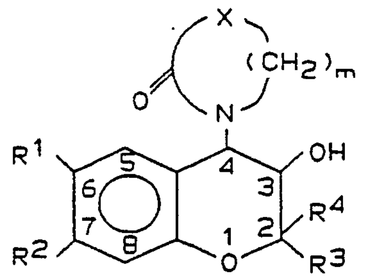

I

in welcher bedeuten:

$R^1$ H, $(C_1\text{-}C_4)$Alkyl, $(C_1\text{-}C_4)$Alkoxy, Halogen, Trifluormethyl, CO-$(C_1\text{-}C_4)$Alkyl, CO-Ar, COOH, Carboxyalkyl-$(C_1\text{-}C_4)$,

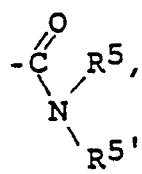

wobei $R^5$ und $R^{5'}$ gleich oder verschieden sind und für H oder $(C_1\text{-}C_2)$Alkyl stehen, C≡N, $NO_2$, $(C_1\text{-}C_4)$Alkyl$SO_r$-, Ar$SO_r$-, wobei r für 0, 1, 2 und Ar für ein aromatisches oder heteroaromatisches System steht, das unsubstituiert oder durch 1 bis 3 gleiche oder verschiedene Reste $(C_1\text{-}C_2)$Alkyl, Halogen, C≡N, $NO_2$, $CF_3$, COOH, Carboxyalkyl-$(C_1\text{-}C_2)$, substituiert ist, steht,

$R^2$ H, OH, $(C_1\text{-}C_2)$Alkoxy, $(C_1\text{-}C_2)$Alkyl oder $(C_1\text{-}C_2)$Alkylcarbonyl,

$R^3$, $R^4$ (gleich oder verschieden) $(C_1\text{-}C_4)$Alkyl,

X eine Kette mit nachfolgender Struktur,

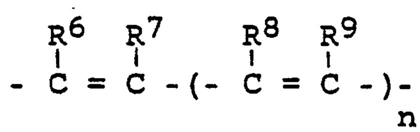

in welcher $R^6$, $R^7$, $R^8$ und $R^9$ gleich oder verschieden sind und für H, $(C_1\text{-}C_4)$Alkyl, Halogen, $NO_2$ stehen,

m null oder 1,

n null oder 1, wobei m und n stets verschieden sind. wobei jedoch für Verbindungen mit m gleich null $R^1$ nur CO-Ar oder Ar$SO_r$ bedeutet.

Unter einem aromatischen System Ar wird vorzugsweise Phenyl, Naphthyl oder Biphenylyl verstanden, ein 5- bis 6-gliedriges heteroaromatisches System Ar ist vorzugsweise ein Rest eines 5- oder 6-gliedrigen O-, N- und/oder S-heterocyclischen Ringes, insbesondere Furyl, Thienyl, Isothiazolyl, Oxazolyl, Isoxazolyl, Pyrazolyl, Imidazolyl, Thiazolyl, Pyridyl, Pyrazinyl, Pyrimidinyl, Pyridazinyl, Thiazinyl.

Unter Halogen wird F, Cl, Br oder J, vorzugsweise F, Cl und Br verstanden.

Die C-Atome 3 und 4 des 3,4-Dihydro-2H-benzo[b]pyransystems (nachfolgend auch kurz "Chromansystem" genannt) der Formel I sind asymmetrisch substituiert. Die Erfindung betrifft dabei nur solche Verbindungen, die an diesen Zentren entgegengesetzte Konfigurationen, also eine "trans-Orientierung" der Substituenten an diesen C-Atomen aufweisen.

Wenn einer der Substituenten $R^1$, $R^2$, $R^3$, $R^4$ und X Asymmetriezentren enthält, oder, falls $R^3$ und $R^4$ ungleich sind (und somit ein asymmetrisches Kohlenstoffatom erzeugen), dann umfaßt die Erfindung sowohl Verbindungen mit S- als auch R-konfigurierten Zentren.

Die Verbindungen können als optische Isomere, als Diastereomere, als Racemate oder als Gemisch

derselben vorliegen.

Bevorzugt sind Verbindungen der Formel I, bei denen $R^1$ für Halogen, C≡N, $NO_2$, Benzoyl und Phenylsulfonyl steht, das unsubstituiert oder wie oben definiert substituiert ist, $R^2$ H oder $(C_1\text{-}C_2)$Alkoxy bedeutet, $R^3$ und $R^4$ wie oben definiert sind, X und m die oben beschriebene Bedeutung haben.

Besonders bevorzugt sind Verbindungen der Formel I, bei denen $R^1$ für Halogen, CN, $NO_2$ und Phenylsulfonyl steht, das unsubstituiert oder durch $(C_1\text{-}C_2)$Alkyl, Cyano, $(C_1\text{-}C_2)$Alkoxy oder Halogen einfach substituiert ist, $R^2$ = H oder $(C_1\text{-}C_2)$Alkoxy bedeutet, $R^3$ und $R^4$ wie oben definiert sind, m 1 ist, n null ist und die Substituenten in X $R^6$ und $R^7$ gleich oder verschieden sein können und für H und $(C_1\text{-}C_4)$-Alkyl stehen.

Ebenfalls bevorzugt sind weiterhin Verbindungen der Formel I, bei denen $R^1$ für Benzoyl und Phenylsulfonyl steht, das unsubstituiert oder durch $(C_1\text{-}C_2)$Alkyl, Cyano, $(C_1\text{-}C_2)$Alkoxy oder Halogen einfach substituiert ist, $R^2$ = H oder $(C_1\text{-}C_2)$Alkoxy bedeutet, $R^3$ und $R^4$ wie oben definiert sind, m null ist, n 1 ist, einer der Substituenten $R^6$ bis $R^9$ in X für $CH_3$, Cl, CN und COOH steht und die restlichen für H stehen.

Besonders bevorzugt sind Verbindungen der Formel I, bei denen $R^1$ für C≡N und Phenylsulfonyl steht, das unsubstituiert oder durch $(C_1\text{-}C_2)$Alkoxy einfach substituiert ist, $R^2$ H oder $(C_1\text{-}C_2)$Alkoxy bedeutet, $R^3$ und $R^4$ wie oben definiert sind, m 1 ist, n null ist und die Substituenten in X $R^6$ und $R^7$ gleich oder verschieden sein können und für H und $(C_1\text{-}C_4)$Alkyl stehen.

Ganz besonders bevorzugt sind jedoch Verbindungen der Formel I, bei denen $R^1$ für Phenylsulfonyl steht, das unsubstituiert oder durch $(C_1\text{-}C_2)$Alkoxy einfach substituiert ist, $R^2$ H oder $(C_1\text{-}C_2)$Alkoxy bedeutet, $R^3$ und $R^4$ wie oben definiert sind, m null ist, n 1 ist, einer der Substituenten $R^6$ bis $R^9$ in X für $CH_3$ oder Cl steht und die restlichen für H stehen.

In der EP-Offenlegungsschrift 273 262 werden in Phenylring durch 1,2-Dihydro-2-oxo-pyrid-1-yl substituierte Chromane vorgeschlagen.

In J. Med. Chem. 1986, 29, 2194-2201 werden Verbindungen beschrieben, die den erfindungsgemäßen Verbindungen strukturell am nächsten stehen. Sie sind dort unter folgenden allgemeinen Formeln zusammengefaßt:

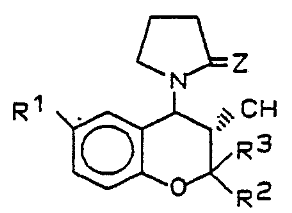

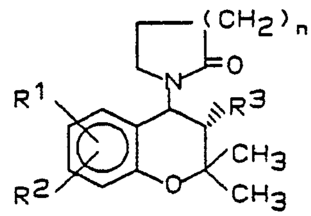

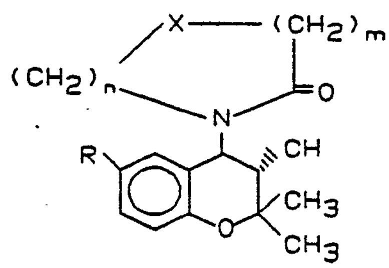

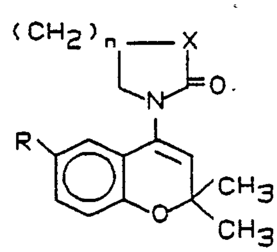

mit $R^1$, $R^2$, $R^3$, Z, n, m, R in den dort aufgeführten Bedeutungen. Ein großer Teil dieser Verbindungen war auch Gegenstand verschiedener Patentanmeldungen, zu nennen sind hierbei EP 0 107 423, EP 0 120 427, EP 0 076 075, EP 0 120 428.

Insbesondere sind in der oben zitierten Literaturstelle solche Verbindungen als besonders wirksam beschrieben worden, die in der 6-Position des 3,4-Dihydro-2H-benzopyransystems eine CN- oder eine $NO_2$-Gruppe tragen, wobei insbesondere dem (±)-6-Cyano-3,4-dihydro-2,2-dimethyl-trans-4-(2-oxo-1-pyrrolidinyl)-2H-benzo[b]pyran-3-ol eine besondere Bedeutung zukommt.

Überraschenderweise führte die Einführung ungesättigter cyclischer Amide in die 4-Position des Chromansystems zu neuen wirksamen Verbindungen mit wertvollen pharmakologischen Eigenschaften. Sie wirken blutdrucksenkend und/oder haben eine relaxierende Wirkung an Organen wie Blase, Darm, Galle, Uterus, Trachea, Ureter. Die erfindungsgemäßen Verbindungen I können somit als Antihypertensiva, als Coronartherapeutika oder als Mittel zur Behandlung der Herzinsuffizienz eingesetzt werden. Sie können aber auch als Spasmolytika für die obengenannten Organe Verwendung finden. Sie können dabei sowohl in

der Human- als auch in der Veterinärmedizin zur Anwendung gelangen.

Die Erfindung betrifft weiterhin ein Verfahren zur Herstellung der Verbindungen I, dadurch gekennzeichnet, daß man

a) Verbindungen der Formel II

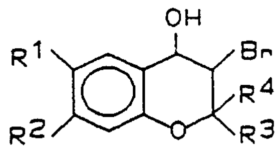

II,

in denen $R^1$ bis $R^4$ wie oben definiert sind, umsetzt mit Lactamen der Formel III

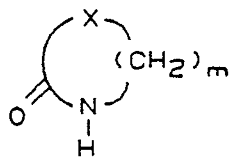

III

oder daß man

b) Verbindungen der Formel IV

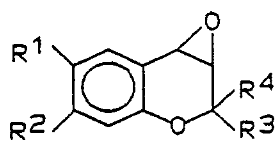

IV,

in denen $R^1$ bis $R^4$ wie oben definiert sind, umsetzt mit Lactamen der Formel III

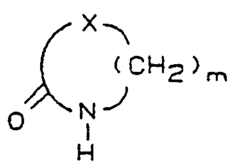

III

oder daß man

c) Verbindungen der Formel IV

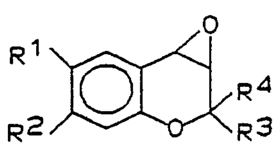

IV,

in denen $R^1$ bis $R^4$ wie oben definiert sind, umsetzt mit den N-Silyllactamen V

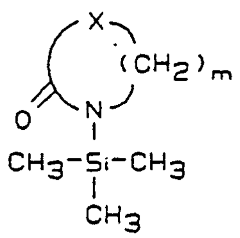

V

oder daß man

d) Verbindungen der Formel IV

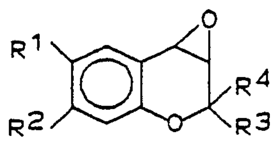

IV,

in denen $R^1$ bis $R^4$ wie oben definiert sind, umsetzt mit den o-Silylderivaten VI

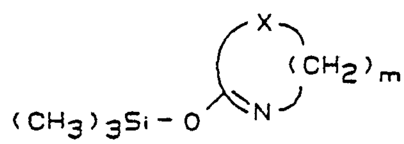

VI

oder daß man

e) Verbindungen der Formel VII

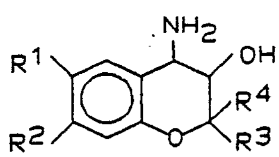

VII,

in denen $R^1$ bis $R^4$ wie oben definiert sind, acyliert zu den Verbindungen VIII

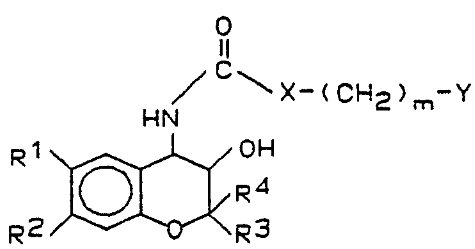

VIII,

in denen Y eine Fluchtgruppe, wie etwa Chlor oder Brom, ist und $R^1$ bis $R^4$ wie oben definiert sind und diese zu den Verbindungen I cyclisiert, oder daß man

f) Verbindungen der Formel IX

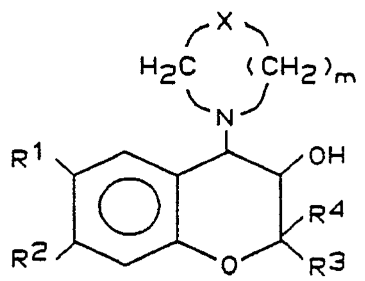

IX,

in denen $R^1$ bis $R^4$ wie oben definiert sind, zu den Verbindungen I oxidiert.

Werden die Verbindungen I nach den Methoden a) oder b) hergestellt, so geschieht dies dadurch, daß man die Verbindungen II oder IV in einem geeigneten Lösemittel, vorzugsweise in dipolar aprotischen Lösungsmitteln wie etwa Dimethylsulfoxid oder THF umsetzt mit den Lactamen III, vorzugsweise unter Einwirkung von Basen, wie etwa Natriumhydrid, Kaliumhydrid, K-tert.butylat, Na-(2-methyl-2-butylat), Lithium-bis-(trimethylsilyl)-amid oder ähnlichen, für Lactam-N-Alkylierungen bekanntermaßen geeigneten Basen. Die Reaktionstemperatur ist dabei im weiten Bereich variierbar; vorzugsweise wird zwischen 0° C und Zimmertemperatur gearbeitet oder bei Temperaturen, die leicht oberhalb der Zimmertemperatur liegen können.

Verbindungen, die nach den Methoden a) oder b) nur schwierig herstellbar sind, können oftmals nach den Verfahren c) und d) sehr gut zugänglich gemacht werden.

Dabei rührt man die Verbindungen IV und die Verbindungen V bzw. VI zusammen in Gegenwart eines Desilylierungsmittels wie Kalium tert.-butylat oder Tetrabutylammoniumfluorid in einem dipolar aprotischen Lösemittel, vorzugsweise jedoch in THF. Es ist auch möglich, die Reaktion ohne Lösungsmittel durchzuführen, wobei gewöhnlich die flüssigen Silylverbindungen V bzw. VI im Überschuß eingesetzt werden, um den Ansatz rührfähig zu halten. Die Temperatur kann dabei in weiten Grenzen variieren. So erhält man in vielen Fällen bereits schon bei Zimmertemperatur die erfindungsgemäßen Verbindungen I, in anderen Fällen erst nach Erhitzen auf 120° C oder sogar darüber.

Lactame der Formel III sind in vielen Fällen bekannt, oder können leicht nach literaturbekannten Methoden hergestellt werden.

Die Silylverbindungen V und VI können leicht nach literaturbekannten Methoden aus den Lactamen der Formel III hergestellt werden, beispielsweise durch Erhitzen mit 1,1,1,3,3,3-Hexamethyldisilazan.

Verbindungen II und IV sind neu. Sie können beispielsweise durch folgenden Syntheseweg hergestellt werden.

Verbindungen der Formel X

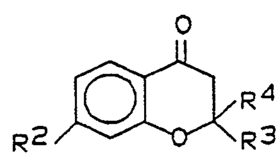

X,

in denen $R^2$, $R^3$ und $R^4$ wie oben definiert sind, werden mit Säurechloriden $ArSO_rCl$ in an sich bekannter Weise nach Art der Friedel-Crafts Acylierung umgesetzt zu Verbindungen der Formel XI

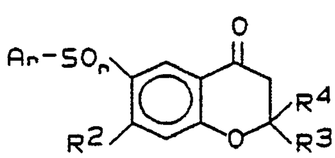

XI,

in denen $R^2$, $R^3$, $R^4$ und Ar wie oben definiert sind.

Verbindungen der Formel X in denen $R^2$, $R^3$ und $R^4$ wie oben definiert sind, werden mit $NaNO_3$ oder N-Bromsuccinimid in konz. Schwefelsäure in an sich bekannter Weise zu Verbindungen der Formel XII und XIII umgesetzt.

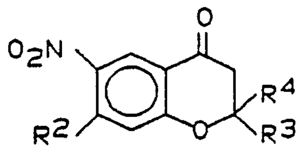

XII

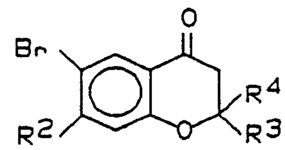

XIII

Die Verbindungen der Formel XII können in an sich bekannter Weise in die entsprechenden Halogenide der Formel XIV überführt werden.

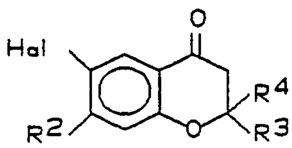

XIV

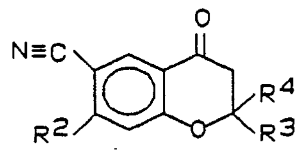

XV

Die Verbindungen der Formel XIII können in an sich bekannter Weise in die entsprechenden Nitrile der Formel XV überführt werden.

Die Verbindungen XI, XII, XIII, XIV und XV werden durch Reduktion unter Standardbedingungen, etwa durch $NaBH_4$ in Methanol oder Ethanol umgesetzt zu den Verbindungen der Formel XVI,

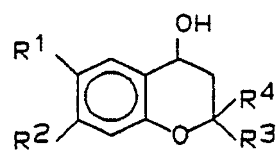

XVI

in denen $R^1$, $R^2$, $R^3$ und $R^4$ wie oben definiert sind.

Die Verbindungen der Formel XVI werden anschließend einer Wasserabspaltung, etwa durch Pyridin/Phosphoroxychlorid, oder p-Toluolsulfonsäure/Toluol unterworfen, wobei Verbindungen XVII entstehen:

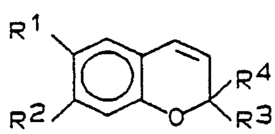

XVII.

Ein besonders zweckmäßiges Verfahren zur Einführung unterschiedlichster Reste $R_1$ in Verbindungen der Formel II und IV besteht darin, daß man Verbindungen der Formel XVII, in denen $R^1$ Br bedeutet, $R^2$, $R^3$ und $R^4$ wie oben definiert sind, bei -78 °C in THF mit Hilfe von 2 Äquivalenten tert.-Butyllithium in 6-Position metalliert und die Aryllithium-Verbindungen in an sich bekannter Weise mit einer Vielzahl von Elektrophilen umsetzt.

Verbindungen der Formel XVII, in denen $R^1$ Br bedeutet, $R^2$, $R^3$ und $R^4$ wie oben definiert sind, können sehr einfach hergestellt werden, indem man Verbindungen der Formel X, in denen $R^2$, $R^3$ und $R^4$ wie oben definiert sind, reduziert, analog der Herstellung der Verbindungen XVI. Man erhält auf diese Weise Verbindungen der Formel XVI, in denen $R^1$ H, $R^2$, $R^3$ und $R^4$ wie oben definiert sind. Die Einführung von $R^1$ = Br in Verbindung der Formel XVI, in denen $R^1$ = H, $R^2$, $R^3$ und $R^4$ wie oben definiert sind, gelingt mit N-Bromsuccinimid in Eisessig.

Man erhält auf diese Weise Verbindungen der Formel XVI, in denen $R^1$ = Br ist und $R^2$, $R^3$ und $R^4$ wie oben definiert sind; diese Verbindungen können wie oben beschrieben in Verbindungen der Formel XVII, in denen $R^1$ = Br und $R^2$, $R^3$ und $R^4$ wie oben definiert sind, überführt werden.

Verbindungen XVII lassen sich nun leicht nach Standardmethoden in die Epoxide IV oder die Bromhydrine II umwandeln.

Steht bei dieser Reaktionssequenz $R^2$ in der Bedeutung von OH, so sind gegebenenfalls Schutzgruppen wie etwa die Acetyl- oder Methylgruppe für die OH-Gruppe notwendig. Diese werden auf geeigneten Stufen, vorzugsweise nach Durchführung der in Verfahren a), b), c), d), e) und f) beschriebenen Umsetzungen, durch gängige Methoden wieder abgespalten.

Chromene der Formel XVII werden in einigen Fällen in an sich bekannter Weise durch thermisch induzierte Cyclisierung der entsprechenden Propargylether XVIII

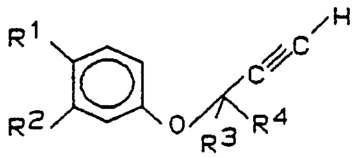

XVIII

hergestellt. Diese wiederum lassen sich in an sich bekannter Weise aus den Phenolen XIX und den Propargylchloriden XX herstellen.

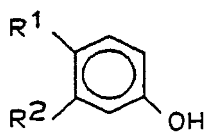

XIX

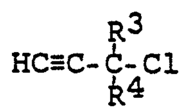

XX

Die Verfahren e) und f) können dann besonders günstig angewandt werden, wenn enantiomerenreine Endprodukte I erwünscht sind.

Verbindungen VII und IX sind im Gegensatz zu Verbindungen I basisch und damit zur Salzbildung mit organischen Säuren befähigt. Durch Kristallisation mit einer geeigneten optisch einheitlichen Säure wie etwa (+) Mandelsäure oder (+) Milchsäure können sie in an sich bekannter Weise enantiomerenrein erhalten werden und in enantiomerenreine Endprodukte I nach den Verfahren e) und f) umgewandelt werden.

Enantiomerenreine Endprodukte I können aber auch aus racemischen Endprodukten I durch gängige Racematspaltungsmethoden wie etwa die chromatographische Trennung unter Verwendung von chiralen Phasen oder die Derivatisierung der racemischen Produkte mit optisch einheitlichen Säurederivaten (Esterbildung über die 3-Hydroxygruppe des Chromansystems) oder mit optisch einheitlichen Isocyanaten (Carbamatbildung über die 3-Hydroxygruppe) aufgespalten werden. Die dabei erhaltenen diastereoisomeren Isocyanate oder Ester lassen sich durch gängige Methoden (Kristallisation oder Chromatographie) trennen und unter Abspaltung der optisch aktiven Hilfsgruppe an der 3-OH-Funktion in die optisch einheitlichen Endverbindungen umwandeln. Als besonders vorteilhaft hat sich hierbei die Trennung der diastereomeren 3-Menthoxyacetate erwiesen.

**Beispiel 1**

trans-3,4-Dihydro-4-(1,2-dihydro-2-oxo-pyrid-1-yl)-2,2-dimethyl- 6-(phenylsulfonyl)-2H-benzo[b]pyran-3-ol

Zu einer Lösung aus 3,16 g (10 mmol) 3,4-Dihydro-2,2-dimethyl-3,4-epoxy-6-(phenylsulfonyl)-2H-benzo-[b]pyran in 4 ml absolutem THF und 3,35 g (20 mmol) 2-Trimethylsilyloxypyridin setzt man unter Argon und unter Kühlung 3,16 g (10 mmol) Tetrabutylammoniumfluorid-trihydrat zu und rührt 60 h bei Zimmertemperatur. Man verdünnt mit Wasser und extrahiert 3mal mit Essigester. Die organische Phase wird mit Wasser und gesättigter Natriumchloridlösung gewaschen, über Natriumsulfat getrocknet, filtriert und im Vakuum eingedampft. Der ölige Rückstand wird an Kieselgel mit Ethylacetat/Cyclohexan 9 : 1 chromatographiert.

Die amorphe weiße Substanz wird in Hochvakuum getrocknet.

| | | | | | | | |
|---|---|---|---|---|---|---|---|
| $C_{22}H_{21}NO_5S$ (429,47) | Ber. | C | 61,52 | H | 5,40 | N | 3,26 |
| $\cdot H_2O$ | Gef. | C | 61,8 | H | 5,1 | N | 3,2 |

Herstellung des Ausgangsmaterials:

3,4-Dihydro-2,2-dimethyl-3,4-epoxy-6-(phenylsulfonyl)-2H-benzo[b]pyran erhält man aus trans-3-Brom-3,4-dihydro-2,2-dimethyl-6-(phenylsulfonyl)-2H-benzo[b]pyran-4-ol mit NaH in DMSO.
Schmp.: 103-105° C

trans-3-Brom-3,4-dihydro-2,2-dimethyl-6-(phenylsulfonyl)-2H-benzo[b]pyran-4-ol erhält man aus 2,2-Dimethyl-6-phenylsulfonyl-2H-chromen und N-Bromsuccinimid in einem 9 : 1 Dimethylsulfoxid/$H_2O$ Gemisch.
Schmp.: 126° C

Das 2,2-Dimethyl-6-phenylsulfonyl-2H-chromen mit dem Smp. 70-71° C wurde nach bekannten Methoden aus 4-Phenylsulfonylphenyl-1,1-dimethylpropargylether hergestellt. Diesen Ether erhielt man ebenfalls in bekannter Weise aus 4-Phenylsulfonylphenol und 3-Methyl-3-chlorbutin.

**Beispiel 2**

trans-4-(5-Chloro-1,2-dihydro-2-oxo-pyrid-1-yl)-3,4-dihydro-6-(phenylsulfonyl)-2H-benzo[b]pyran-3-ol

3,16 g (10 mmol) 3,4-Dihydro-2,2-dimethyl-3,4-epoxy-6-(phenylsulfonyl)-2H-benzo[b]pyran werden in 4 ml abs. THF gelöst und unter Argon 4,00 g (20 mmol) 5-Chlor-2-trimethylsilyloxy-pyridin zugesetzt. Dann wird unter Kühlung 3,16 g (20 mmol) Tetrabutylammoniumfluoridtrihydrat fest zugegeben und ca. 60 h bei Zimmertemperatur gerührt. Zur Aufarbeitung versetzt man mit Wasser und extrahiert 3mal mit Essigester. Die organische Phase wird mit Wasser und konz. wäßriger Natriumchloridlösung gewaschen. Nach Trocknung über Magnesiumsulfat, Filtration und Abdampfen des Lösungsmittels im Vakuum erhält man einen weißen Schaum, der sich in Butylacetat kristallisieren läßt.

Man erhält weiße Kristalle vom Schmp. 218-220° C.

| | | | | | | | |
|---|---|---|---|---|---|---|---|
| $C_{22}H_{20}ClNO_5S$ (445,90) | Ber. | C | 59,26 | H | 4,52 | N | 3,14 |
| | Gef. | C | 59,1 | H | 4,5 | N | 3,1 |

Herstellung des Ausgangsmaterials:

3,4-Dihydro-2,2-dimethyl-3,4-epoxy-6-(phenylsulfonyl)-2H-benzo[b]pyran, siehe unter Beispiel 1.

5-Chlor-2-trimethylsilyloxy-pyridin erhält man durch Erhitzen von 5-Chlor-2-hydroxy-pyridin mit 1,1,1,3,3,3-Hexamethyldisilazan auf 100 bis 110° C und anschließende Destillation im Vakuum.

**Beispiel 3**

trans-3,4-Dihydro-4-(1,2-dihydro-2-oxo-pyrid-1-yl)-2,2-dimethyl-6-(2-methoxyphenylsulfonyl)-2H-benzo[b]-pyran-3-ol

3,5 g (0,0082 Mol) trans-3-Brom-3,4-dihydro-2,2-dimethyl-6-(2-methoxyphenylsulfonyl)-2H-benzo[b]-pyran-4-ol werden zu einer Suspension von 0,6 g NaH (80 %ig in Öl) (0,0246 Mol) und 3,1 g 2-Hydroxypyridin in 50 ml Dimethylsulfoxid gegeben. Die Mischung wird auf 60° erwärmt und 3 h gerührt. Die Mischung wird auf Eiswasser gegossen und der weiße Niederschlag abgesaugt und an der Luft getrocknet. Chromatographie an Kieselgel mit Methanol/Essigester 1 : 10 liefert das Produkt als weißes, amorphes Pulver.

| | | | | | | | |
|---|---|---|---|---|---|---|---|
| $C_{23}H_{23}NO_6S$ (441,49) | Ber. | C | 62,57 | H | 5,25 | N | 3,17 |
| | Gef. | C | 61,8 | H | 5,3 | N | 2,9 |

Herstellung des Ausgangsmaterials:

trans-3-Brom-3,4-dihydro-2,2-dimethyl-6-(2-methoxyphenylsulfonyl)-2H-benzo[b]pyran-4-ol wird analog Beispiel 1 hergestellt.

**Beispiel 4**

trans-3,4-Dihydro-4-(1,2-dihydro-2-oxo-pyrid-1-yl)-2,2-dimethyl-7-methoxy-6-phenylsulfonyl-2H-benzo[b]-pyran-3-ol

Herstellung analog Beispiel 3. Die Reinigung erfolgt über Chromatographie an Kieselgel mit Toluol/Essigester 4 : 1. Weiße Kristalle vom Schmp. 228-29 °C.

Herstellung des Ausgangsmaterials:

trans-3-Brom-3,4-dihydro-2,2-dimethyl-7-methoxy-6-phenylsulfonyl-2H-benzo[b]pyran-4-ol erhält man aus 2,2-Dimethyl-7-methoxy-6-phenylsulfonyl-2H-chroman und N-Bromsuccinimid in einem 9 : 1 Dimethylsulfoxid/$H_2O$ Gemisch.
Schmp.: 202-203 °C.

2,2-Dimethyl-7-methoxy-6-phenylsulfonyl-2H-chromen erhält man aus 2,2-Dimethyl-4-hydroxy-7-methoxy-6-phenylsulfonylchromen mit Pyridin/Phosphoroxychlorid in Benzol.
Schmp.: 140-41 °C.

2,2-Dimethyl-4-hydroxy-7-methoxy-6-phenylsulfonyl-chroman erhält man aus 2,2-Dimethyl-7-methoxy-6-phenylsulfonylchroman-4-on mit Natriumborhydrid in Methanol.
Schmp.: 146-147 °C.

2,2-Dimethyl-7-methoxy-6-phenylsulfonyl-chroman-4-on erhält man aus Phenylsulfonylchlorid, 2,2-Dimethyl-7-methoxychroman-4-on und Aluminiumchlorid in Methylenchlorid.
Schmp.: 223-25 °C.

**Beispiel 5**

trans-3,4-Dihydro-2,2-dimethyl-4-(3-ethyl-4-methyl-2-oxo-3-pyrrolin-1-yl)-6-(phenylsulfonyl)-2H-benzo[b]-pyran-3-ol

3,80 g (12,02 mmol) 3,4-Dihydro-2,2-dimethyl-3,4-epoxy-6-(phenylsulfonyl)-2H-benzo[b]pyran und 1,50 g (12,02 mmol) 3-Ethyl-4-methyl-2-oxo-3-pyrrolin werden in 5 ml absolutem THF gelöst, und unter Argon werden 12,02 ml Lithium-bis-(trimethylsilyl)-amid (1molare molare Lösung in THF) zugespritzt. Man rührt 72 h bei Zimmertemperatur, verdünnt anschließend mit Wasser. Das sich abscheidende zähe Öl wird 2mal in Butylacetat kristallisiert. Man erhält weiße Kristalle vom Schmp 128-135 °C.

| | | | | | | | |
|---|---|---|---|---|---|---|---|
| $C_{24}H_{27}NO_5S$ (441,52) | Ber. | C | 63,98 | H | 6,26 | N | 3,11 |
| | Gef. | C | 63,8 | H | 6,2 | N | 3,1 |

Herstellung des Ausgangsmaterials:

3,4-Dihydro-2,2-dimethyl-3,4-epoxy-6-(phenylsulfonyl)-2H-benzo[b]pyran, siehe unter Beispiel 1.
3-Ethyl-4-methyl-2-oxo-3-pyrrolin ist literaturbekannt.

**Beispiel 6**

trans-3,4-Dihydro-2,2-dimethyl-4-(3,4-dimethyl-2-oxo-3-pyrrolin-1-yl)-6-(phenylsulfonyl)-2H-benzo[b]-pyran-3-ol

Die Verbindung kann analog Beispiel 5 hergestellt werden. Man erhält nach Kristallisation in Ethylacetat/Cyclohexan weiße Kristalle vom Schmp. 191-193 °C.

| | | | | | | | |
|---|---|---|---|---|---|---|---|
| $C_{23}H_{25}NO_5S$ (436,50) | Ber. | C | 63,28 | H | 5,87 | N | 3,21 |
| •0,5 $H_2O$ | Gef. | C | 63,3 | H | 6,0 | N | 3,2 |

Herstellung des Ausgangsmaterials

3,4-Dihydro-2,2-dimethyl-3,4-epoxy-6-(phenylsulfonyl)-2H-benzo[b]pyran, siehe unter Beispiel 1.
3,4-Dimethyl-2-oxo-3-pyrrolin ist literaturbekannt.

**Beispiel 7**

trans-6-Cyano-3,4-dihydro-2,2-dimethyl-4-(3-ethyl-4-methyl-2-oxo-3-pyrrolin-1-yl)-2H-benzo[b]pyran-3-ol

Zu einer Lösung von 14,4 g (0,072 Mol) trans-3-Brom-6-cyano-3,4-dihydro-2,2-dimethyl-2H-benzo[b]-pyran-4-ol in 200 ml DMSO werden 2,4 g (0,08 Mol) 80 %iges NaH eingetragen. Nach einstündigem Rühren bei 20 °C werden 3,6 g (0,12 Mol) 80 %iges NaH und 15 g (0,12 Mol) 3-Ethyl-4-methyl-2-oxo-3-pyrrolin eingetragen. Nach dreistündigem Rühren bei 40 °C wird das Reaktionsgemisch in Eiswasser eingetragen, abgesaugt, getrocknet und über eine Kieselgelsäule mit Methylenchlorid/Methanol (95 : 5) chromatographiert. Das so gewonnene Produkt kristallisiert man aus wenig Ethanol um.
Kristalle vom Schmp. 207-208 °C.

| | | | | | | | |
|---|---|---|---|---|---|---|---|
| $C_{19}H_{22}N_2O_3$ (326,41) | Ber. | C | 69,92 | H | 6,80 | N | 8,58 |
| | Gef. | C | 70,0 | H | 6,7 | N | 8,6 |

Herstellung des Ausgangsmaterials:

trans-3-Brom-6-cyano-3,4-dihydro-2,2-dimethyl-2H-benzo[b]pyran-4-ol und 3-Ethyl-4-methyl-2-oxo-3-pyrrolin sind literaturbekannt.

**Beispiel 8**

trans-6-Cyano-3,4-dihydro-2,2-dimethyl-4-(3,4-dimethyl-2-oxo-3-pyrrolin-1-yl)-2H-benzo[b]pyran-3-ol

Die Verbindung kann analog Beispiel 5 hergestellt werden. Man erhält nach Kristallisation in Wasser/Ethanol Kristalle vom Schmp. 217-219 °C.

| | | | | | | | |
|---|---|---|---|---|---|---|---|
| $C_{18}H_{20}N_2O_3$ (312,35) | Ber. | C | 69,21 | H | 6,45 | N | 8,92 |
| | Gef. | C | 68,9 | H | 6,2 | N | 8,9 |

Herstellung des Ausgangsmaterials:

6-Cyano-3,4-dihydro-2,2-dimethyl-3,4-epoxy-2H-benzo[b]pyran und 2-Trimethylsilyloxy-pyridin sind literaturbekannt.

**Beispiel 9**

trans-3,4-Dihydro-4-(1,2-dihydro-5-nitro-2-oxo-pyrid-1-yl)-2,2-dimethyl-6-phenylsulfonyl-2H-benzo[b]pyran-3-ol

4,00 g (12,6 mmol) 3,4-Dihydro-2,2-dimethyl-3,4-epoxy-6-(phenylsulfonyl)-2H-benzo[b]pyran und 1,77 g (12,6 mmol) 2-Hydroxy-5-nitropyridin werden mit wenig absolutem THF angeteigt und dann unter Argon tropfenweise mit 16,7 ml (12,6 mmol) einer 1 M-Lösung von Lithium-bis-trimethylsilylamid unter Rühren und Eiskühlung versetzt. Man rührt ca. 72 h bei Zimmertemperatur nach, hydrolysiert und extrahiert 3 mal mit Essigester. Die vereinigten organischen Extrakte werden mit Wasser und gesättigter Natriumchloridlösung gewaschen und über Natriumsulfat getrocknet. Nach Abdampfen des Lösungsmittels im Vakuum wird der Rückstand über Kieselgel chromatographiert (Essigester/Cyclohexan 1:1). Die Verbindung kann in Essigester kristallisiert werden.
Schmp. 212-214 °C

| | | | | | | | |
|---|---|---|---|---|---|---|---|
| $C_{22}H_{20}N_2O_7S$ (456,48) | Ber. | C | 57,8 | H | 4,4 | N | 6,1 |
| | Gef. | C | 57,7 | H | 4,2 | N | 6,2 |

Herstellung des Ausgangsmaterials siehe unter Beispiel 1.

**Beispiel 10**

trans-3,4-Dihydro-4-(1,2-dihydro-2-oxo-pyrid-1-yl)-2,2-dimethyl-6-(2-trifluormethyl-benzoyl)-2H-benzo[b]-pyran-3-ol

5,15 g (12 mmol) trans-3-Brom-3,4-dihydro-2,2-dimethyl-6-(2-trifluormethyl-benzoyl)-2H-benzo[b]pyran-4-ol werden unter Argon in 6,02 g (36 mmol) 2-Trimethylsilyloxypyridin suspendiert und unter Rühren 9,46 g (30 mmol) Tetrabutylammoniumfluorid-trihydrat zugesetzt. Man rührt ca. 15 Std. bei 55 °C nach, hydrolisiert, extrahiert 3 mal mit Essigester. Die vereinigten Essigesterextrakte werden mit Wasser und gesättigter Natriumchloridlösung gewaschen, mit Natriumsulfat getrocknet und das Lösungsmittel im Vakuum abgedampft. Der schaumige Rückstand wird mit Diisopropylether verrieben und abgesaugt. Die Kristalle werden im Hochvakuum getrocknet.
Schmp. 184-186 °C

| | | | | | | | |
|---|---|---|---|---|---|---|---|
| $C_{24}H_{20}F_3NO_4$ (443,43) | Ber. | C | 65,0 | H | 4,54 | N | 3,16 |
| | Gef. | C | 65,3 | H | 4,6 | N | 3,1 |

Herstellung des Ausgangsmaterials:

Die Umsetzung von 2-Hydroxyacetophenon mit Aceton in Gegenwart von Pyrrolidin analog Synthesis 886, 1978 ergibt 2,2-Dimethyl-4-chromanon vom Schmelzpunkt 80-85 °C. Aus 2,2-Dimethyl-4-chromanon erhält man durch Redaktion mit Natriumborhydrid in Ethanol 2,2-Dimethyl-4-hydroxy-chroman (Öl).

Durch Umsetzung von 2,2-Dimethyl-4-hydroxy-chroman mit N-Bromsuccinimid in Eisessig erhält man 6-Brom-2,2-dimethyl-4-hydroxy-chroman vom Schmp. 97-99 °C.
Durch azeotrope Wasserabspaltung in Toluol unter Zusatz katalytischer Mengen p-Toluolsulfonsäure erhält man aus 6-Brom-2,2-dimethyl-4-hyroxy-chroman das ölige 6-Brom-2,2-dimethyl-2H-chroman.
6-Brom-2,2-dimethyl-2H-chromen wird mit 2 Äquivalenten tert.-Butyllithium bei -78 °C metalliert und

anschließend mit 2-Trifluormethylbenzaldehyd umgesetzt. Dabei erhält man 2,2-Dimethyl-6-(2-trifluormethyl-hydroxybenzyl)-2H-chromen, das als Öl anfällt.
2,2-Dimethyl-6-(2-trifluormethyl-hydroxybenzyl)-2H-chromen wird durch Oxidation mit Pyridinium-chlorochromat in Methylenchlorid in 2,2-Dimethyl-6-(2-trifluormethyl-benzoyl)-2H-chroman überführt, aus dem wie in den vorhergehenden Beispielen beschrieben das ölige trans-3-Brom-3,4-dihydro-2,2-dimethyl-6-(2-trifluormethyl-benzoyl)-2H-benzo[b]pyran-4-ol erhalten wird.

**Beispiel 11**

In analoger Weise wie unter Beispiel 10 beschrieben erhält man trans-3,4-Dihydro-4-(1,2-dihydro-2-oxo-pyrid-1-yl)-2,2-dimethyl-6-(2-fluor-benzoyl)-2H-benzo[b]pyran-3-ol vom Schmp. 241-243 °C.

| | | | | | | | |
|---|---|---|---|---|---|---|---|
| $C_{23}H_{20}FNO_4$ (393,93) | Ber. | C | 70,21 | H | 5,12 | N | 3,56 |
| | Gef. | C | 70,1 | H | 5,2 | N | 3,5 |

## Ansprüche

1. Ungesättigte 3,4-Dihydro-2H-benzo[b]-pyrane der Formel I

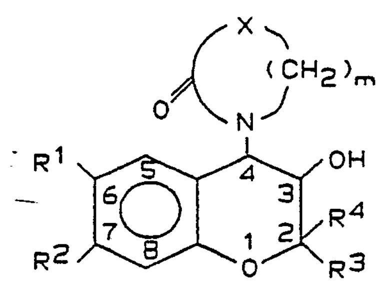

I

in welcher bedeuten:
$R^1$ H, $(C_1-C_4)$Alkyl, $(C_1-C_4)$Alkoxy, Halogen, Trifluormethyl, CO-$(C_1-C_4)$Alkyl, CO-Ar, COOH, Carboxyalkyl-$(C_1-C_4)$,

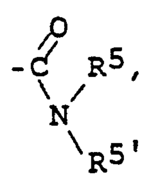

wobei $R^5$ und $R^{5'}$ gleich oder verschieden sind und für H oder $(C_1-C_2)$Alkyl stehen,
C≡N, $NO_2$, $(C_1-C_4)$Alkyl$SO_r$-, Ar$SO_r$-, wobei r für 0, 1, 2 und Ar für ein aromatisches oder heteroaromatisches System steht, das unsubstituiert oder durch 1 bis 3 gleiche oder verschiedene Reste $(C_1-C_2)$Alkyl, Halogen, C≡N, $NO_2$, $CF_3$, COOH, Carboxyalkyl-$(C_1-C_2)$, substituiert ist, steht,
$R^2$ H, OH, $(C_1-C_2)$Alkoxy, $(C_1-C_2)$Alkyl oder $(C_1-C_2)$Alkylcarbonyl,
$R^3$, $R^4$ (gleich oder verschieden) $(C_1-C_4)$Alkyl,
X eine Kette mit nachfolgender Struktur,

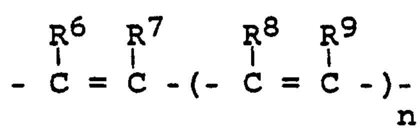

in welcher $R^6$, $R^7$, $R^8$ und $R^9$ gleich oder verschieden sind und für H, $(C_1-C_4)$Alkyl, Halogen, $NO_2$ stehen,
m null oder 1,
n null oder 1, wobei m und n stets verschieden sind. wobei jedoch für Verbindungen mit m gleich null $R^1$ nur CO-Ar oder $ArSO_r$ bedeutet.

2. Verbindung I nach Anspruch 1, dadurch gekennzeichnet, daß die Substituenten und Indices folgende Bedeutung haben:
$R^1$ F, Cl, Br, J, CN, $NO_2$, Benzoyl, Phenylsulfonyl, wobei der Phenylkern unsubstituiert oder wie in Anspruch 1 definiert substituiert ist,
$R^2$ H, $(C_1-C_2)$Alkoxy,
$R^3$, $R^4$, X und m wie im Anspruch 1 definiert.

3. Verbindung I nach Anspruch 1, dadurch gekennzeichnet, daß die Substituenten und Indices folgende Bedeutung haben:
$R^1$ F, Cl, Br, J, CN, $NO_2$, Phenylsulfonyl, wobei der Phenylkern unsubstituiert oder durch $(C_1-C_2)$Alkyl, CN, $(C_1-C_2)$Alkoxy oder F, Cl, Br, J einfach substituiert ist,
$R^2$ H, $(C_1-C_2)$Alkoxy,
$R^3$ und $R^4$ wie in Anspruch 1 definiert,
m 1
X wie in Anspruch 1 definiert mit n null, $R^6$ und $R^7$ (gleich oder verschieden) H, $(C_1-C_4)$Alkyl.

4. Verbindung I nach Anspruch 1, dadurch gekennzeichnet, daß die Substituenten und Indices folgende Bedeutung haben:
$R^1$ Phenylsulfonyl, Benzoyl,
$R^2$ wie in Anspruch 3 definiert,
$R^3$ und $R^4$ wie in Anspruch 1 definiert,
m null,
X wie in Anspruch 1 definiert, aber mit
n 1
einem der Substituenten $R^6$ bis $R^9$ gleich $CH_3$, Cl, CN, COOH und den anderen drei gleich H.

5. Verbindung I nach Anspruch 1, dadurch gekennzeichnet, daß die Substituenten und Indices folgende Bedeutung haben:
$R^1$ CN, Phenylsulfonyl, unsubstituiert oder einfach substituiert durch $(C_1-C_2)$Alkoxy, $R^2$ H, $(C_1-C_2)$Alkoxy,
$R^3$ und $R^4$ wie in Anspruch 1 definiert,
m 1
X wie in Anspruch 1 definiert, jedoch mit n null, $R^6$ und $R^7$ (gleich oder verschieden) H, $(C_1-C_4)$Alkyl,

6. Verbindung I nach Anspruch 1, dadurch gekennzeichnet, daß die Substituenten und Indices folgende Bedeutung haben:
$R^1$ Phenylsulfonyl, Ar-CO, wobei der Phenylkern jeweils unsubstituiert oder durch $(C_1-C_2)$Alkoxy einfach substituiert ist,
$R^2$ H, $(C_1-C_2)$Alkoxy,
$R^3$ und $R^4$ wie in Anspruch 1 definiert,
m null,
einer der Substituenten $R^6$ bis $R^9$ in X Cl oder $CH_3$ und die anderen H.

7. Verfahren zum Herstellen einer Verbindung I nach Anspruch 1, dadurch gekennzeichnet, daß man
a) Verbindungen der Formel II

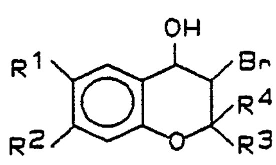

II,

in denen $R^1$ bis $R^4$ wie oben definiert sind, umsetzt mit Lactamen der Formel III

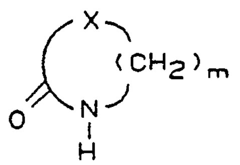

III

oder daß man

b) Verbindungen der Formel IV

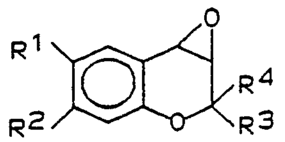

IV,

in denen $R^1$ bis $R^4$ wie oben definiert sind, umsetzt mit Lactamen der Formel III

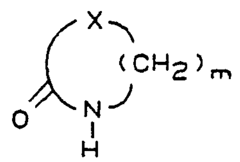

III

oder daß man

c) Verbindungen der Formel IV

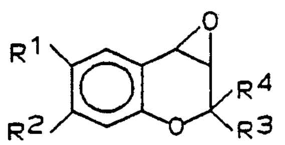

IV,

in denen $R^1$ bis $R^4$ wie oben definiert sind, umsetzt mit den N-Silyllactamen V

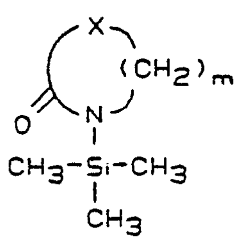

V

oder daß man

d) Verbindungen der Formel IV

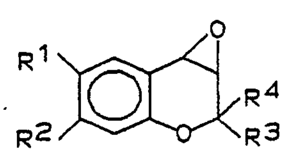

IV,

in denen $R^1$ bis $R^4$ wie oben definiert sind, umsetzt mit den o-Silylderivaten VI

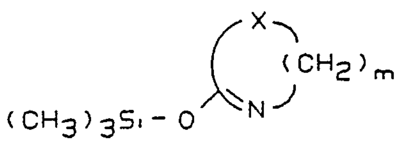

VI

oder daß man

e) Verbindungen der Formel VII

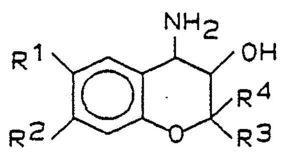

VII,

in denen $R^1$ bis $R^4$ wie oben definiert sind, acyliert zu den Verbindungen VIII

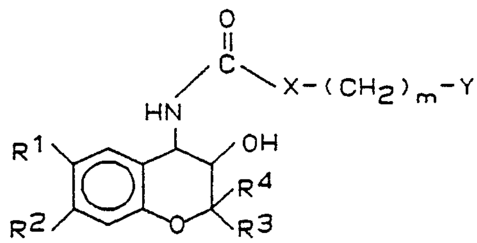

VIII,

in denen Y eine Fluchtgruppe, wie etwa Chlor oder Brom, ist und $R^1$ bis $R^4$ wie oben definiert sind und diese zu den Verbindungen I cyclisiert, oder daß man

f) Verbindungen an der Formel IX

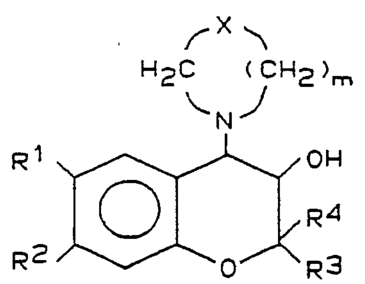

IX,

in denen $R^1$ bis $R^4$ wie oben definiert sind, zu den Verbindungen I oxidiert.

8. Verbindung I nach Anspruch 1 als Heilmittel zum Senken des Blutdrucks oder zum Relaxieren von Blase, Darm, Galle, Uterus, Trachea oder Ureter.

9. Verwendung einer Verbindung nach Anspruch 1 zur Herstellung eines Heilmittels zum Relaxieren von Blase, Darm, Galle, Uterus, Trachea oder Ureter.

Patentansprüche für folgende Vertragsstaaten: ES, GR

1. Ungesättigte 3,4-Dihydro-2H-benzo[b]-pyrane der Formel I

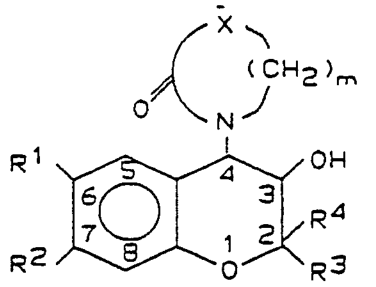

I

in welcher bedeuten:

$R^1$ H, $(C_1\text{-}C_4)$Alkyl, $(C_1\text{-}C_4)$Alkoxy, Halogen, Trifluormethyl, CO-$(C_1\text{-}C_4)$Alkyl, CO-Ar, COOH, Carboxyalkyl-$(C_1\text{-}C_4)$,

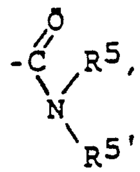

wobei $R^5$ und $R^{5'}$ gleich oder verschieden sind und für H oder $(C_1\text{-}C_2)$Alkyl stehen,

C≡N, $NO_2$, $(C_1\text{-}C_4)$Alkyl$SO_r$-, Ar$SO_r$-, wobei r für 0, 1, 2 und Ar für ein aromatisches oder heteroaromatisches System steht, das unsubstituiert oder durch 1 bis 3 gleiche oder verschiedene Reste $(C_1\text{-}C_2)$Alkyl, Halogen, C≡N, $NO_2$, $CF_3$, COOH, Carboxyalkyl-$(C_1\text{-}C_2)$, substituiert ist, steht,

$R^2$ H, OH, $(C_1\text{-}C_2)$Alkoxy, $(C_1\text{-}C_2)$Alkyl oder $(C_1\text{-}C_2)$Alkylcarbonyl,

$R^3$, $R^4$ (gleich oder verschieden) $(C_1\text{-}C_4)$Alkyl,

X eine Kette mit nachfolgender Struktur,

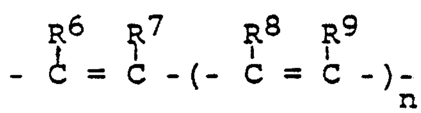

in welcher $R^6$, $R^7$, $R^8$ und $R^9$ gleich oder verschieden sind und für H, $(C_1\text{-}C_4)$Alkyl, Halogen, $NO_2$ stehen,

m null oder 1,

n null oder 1, wobei m und n stets verschieden sind, wobei jedoch für Verbindungen mit m gleich null $R^1$ nur CO-Ar oder Ar$SO_r$ bedeutet,

dadurch gekennzeichnet, daß man

a) Verbindungen der Formel II

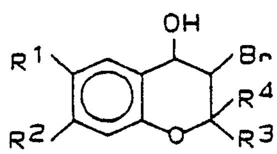

II,

in denen $R^1$ bis $R^4$ wie oben definiert sind, umsetzt mit Lactamen der Formel III

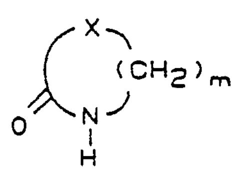

III

oder daß man

b) Verbindungen der Formel IV

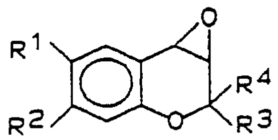

IV,

in denen $R^1$ bis $R^4$ wie oben definiert sind, umsetzt mit Lactamen der Formel III

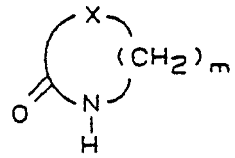

III

oder daß man

c) Verbindungen der Formel IV

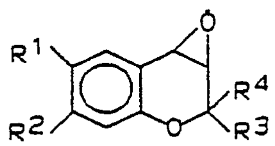

IV,

in denen $R^1$ bis $R^4$ wie oben definiert sind, umsetzt mit den N-Silyllactamen V

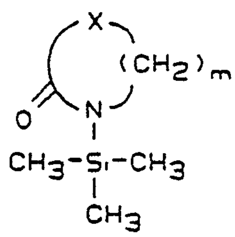

V

oder daß man

d) Verbindungen der Formel IV

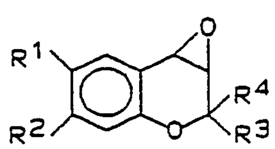

IV,

in denen $R^1$ bis $R^4$ wie oben definiert sind, umsetzt mit den o-Silylderivaten VI

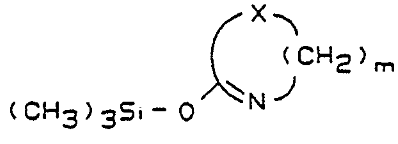

VI

oder daß man

e) Verbindungen der Formel VII

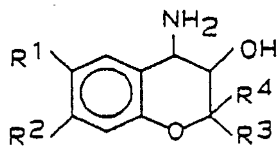

VII,

in denen $R^1$ bis $R^4$ wie oben definiert sind, acyliert zu den Verbindungen VIII

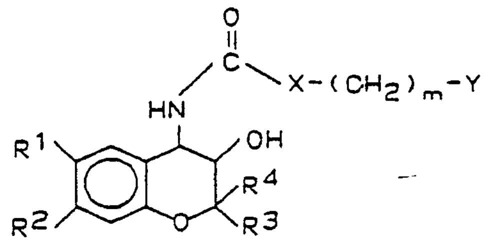

VIII,

in denen Y eine Fluchtgruppe, wie etwa Chlor oder Brom, ist und $R^1$ bis $R^4$ wie oben definiert sind und diese zu den Verbindungen I cyclisiert, oder daß man

f) Verbindungen an der Formel IX

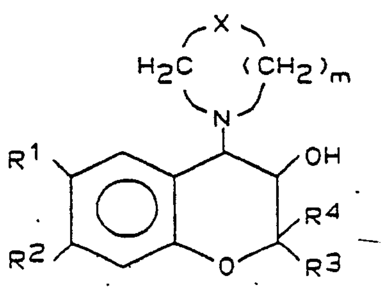

IX,

in denen $R^1$ bis $R^4$ wie oben definiert sind, zu den Verbindungen I oxidiert.

2. Verfahren nach Anspruch 1, dadurch gekennzeichnet, daß die Substituenten und Indices folgende Bedeutung haben:
$R^1$ F, Cl, Br, J, CN, $NO_2$, Benzoyl, Phenylsulfonyl, wobei der Phenylkern unsubstituiert oder wie in Anspruch 1 definiert substituiert ist,
$R^2$ H, $(C_1$-$C_2)$Alkoxy,
$R^3$, $R^4$, X und m wie im Anspruch 1 definiert.

3. Verfahren nach Anspruch 1, dadurch gekennzeichnet, daß die Substituenten und Indices folgende Bedeutung haben:
$R^1$ F, Cl, Br, J, CN, $NO_2$, Phenylsulfonyl, wobei der Phenylkern unsubstituiert oder durch $(C_1$-$C_2)$Alkyl, CN, $(C_1$-$C_2)$Alkoxy oder F, Cl, Br, J einfach substituiert ist,
$R^2$ H, $(C_1$-$C_2)$Alkoxy,
$R^3$ und $R^4$ wie in Anspruch 1 definiert,
m 1
X wie in Anspruch 1 definiert mit n null, $R^6$ und $R^7$ (gleich oder verschieden) H, $(C_1$-$C_4)$Alkyl.

4. Verfahren nach Anspruch 1, dadurch gekennzeichnet, daß die Substituenten und Indices folgende Bedeutung haben:
$R^1$ Phenylsulfonyl, Benzoyl,
$R^2$ wie in Anspruch 3 definiert,
$R^3$ und $R^4$ wie in Anspruch 1 definiert,
m null,
X wie in Anspruch 1 definiert, aber mit
n 1
einem der Substituenten $R^6$ bis $R^9$ gleich $CH_3$, Cl, CN, COOH und den anderen drei gleich H.

5. Verfahren nach Anspruch 1, dadurch gekennzeichnet, daß die Substituenten und Indices folgende Bedeutung haben:
$R^1$ Phenylsulfonyl, unsubstituiert oder einfach substituiert durch $(C_1-C_2)$Alkoxy,
$R^2$ H, $(C_1-C_2)$Alkoxy,
$R^3$ und $R^4$ wie in Anspruch 1 definiert,
m 1
X wie in Anspruch 1 definiert, jedoch mit n null, $R^6$ und $R^7$ (gleich oder verschieden) H, $(C_1-C_4)$Alkyl,

6. Verbindung I nach Anspruch 1, dadurch gekennzeichnet, daß die Substituenten und Indices folgende Bedeutung haben:
$R^1$ Phenylsulfonyl, ArCO, wobei der Phenylkern jeweils unsubstituiert oder durch $(C_1-C_2)$Alkoxy einfach substituiert ist,
$R^2$ H, $(C_1-C_2)$Alkoxy,
$R^3$ und $R^4$ wie in Anspruch 1 definiert,
m null,
einer der Substituenten $R^6$ bis $R^9$ in X, Cl oder $CH_3$ und die anderen H.

7. Verfahren zum Herstellen eines Heilmittels zum Senken des Blutdrucks oder zum Relaxieren von Blase, Darm, Galle, Uterus, Trachea oder Ureter, dadurch gekennzeichnet, daß man eine wirksame Menge einer Verbindung I nach Anspruch 1 mit pharmazeutisch üblichen Zuschlagstoffen mischt und in eine Darreichungsform überführt.

Europäisches Patentamt

# EUROPÄISCHER RECHERCHENBERICHT

Nummer der Anmeldung

EP 89 10 5352

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | EP-A-0 076 075 (BEECHAM GROUP PLC) * Seite 1, Anspruch 1; Seite 7, Ansprüche 19,20 * --- | | C 07 D 405/04<br>A 61 K 31/535 |
| A | EP-A-0 091 748 (BEECHAM GROUP PLC) * Seite 19, Beispiel 1; Seite 20, Beispiel 2; Seite 1, Anspruch 1; Seite 4, Ansprüche 12,13 * --- | | |
| A,D | EP-A-0 120 428 (BEECHAM GROUP PLC) * Seite 22, Zeilen 19-24; Seite 1, Anspruch 1; Seite 2, Ansprüche 2,3; Seite 3, Anspruch 8; Seite 11, Anspruch 32 * --- | | |
| A,D | JOURNAL OF MEDICINAL CHEMISTRY Band 29, Nr. 11, 1986, Seiten 2194-2201; J. M. EVANS et al.: "Synthesis and Antihypertensive Activity of 4-(Cyclic amido)-2H-1-benzopyrans" * Seiten 2195-2197 * ----- | | RECHERCHIERTE SACHGEBIETE (Int. Cl.4)<br>C 07 D 311/00<br>C 07 D 405/00 |

Der vorliegende Recherchenbericht wurde für alle Patentansprüche erstellt

| Recherchenort | Abschlußdatum der Recherche | Prüfer |
|---|---|---|
| BERLIN | 20-07-1989 | KYRIAKAKOU G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument